# EUROPÄISCHE PATENTSCHRIFT

(11) **EP 3 212 030 B1**
(45) Veröffentlichungstag und Bekanntmachung des Hinweises auf die Patenterteilung: **28.09.2022**
(21) Anmeldenummer: 15790498.8
(22) Anmeldetag: 28.10.2015
(51) Int. Cl.: A44C 15/00, A44C 17/00, H01L 31/054, H01L 31/042

(54) **DEKORATIVER VERBUNDKÖRPER MIT SOLARZELLE**
DECORATIVE COMPOSITE BODY COMPRISING A SOLAR CELL
CORPS COMPOSITE DÉCORATIF COMPORTANT UNE CELLULE SOLAIRE

(30) Priorität: 31.10.2014 EP 14191386
(43) Veröffentlichungstag der Anmeldung: 06.09.2017
(73) Patentinhaber: D. Swarovski KG, 6112 Wattens (AT)
(72) Erfinder: MAIR, Mathias, A-6176 Völs (AT); LEXER, Franz, A-6094 Axams (AT); KURTZE, Andreas, A-6122 Fritzens (AT)
(74) Vertreter: Moore, Michael Richard
(86) Internationale Anmeldenummer: PCT/EP2015/074974
(87) Internationale Veröffentlichungsnummer: WO 2016/066680

(56) Entgegenhaltungen:
- EP-A1- 2 458 457
- WO-A2-2010/033859
- CN-A- 101 606 769
- DE-A1- 2 444 705
- DE-A1- 4 433 623
- GB-A- 2 472 805
- JP-A- H0 774 380
- JP-A- S6 176 400
- JP-A- 2010 151 511
- US-A- 5 690 412
- US-A- 5 853 826
- US-A1- 2014 260 424
- US-B1- 6 197 428

## Beschreibung

### GEBIET DER ERFINDUNG

Die Erfindung betrifft ein Schmuckelement, welches einen facettierten, transparenten Körper umfassend konvex gekrümmte Bereiche, eine wellenlängenselektive Schicht und ein photovoltaisches Element enthält. Das Schmuckelement ist zur Energieversorgung, unter anderem im Bereich tragbarer Elektronik, geeignet.

### STAND DER TECHNIK

Facettierte Schmucksteine wurden bisher nahezu ausschließlich für rein ästhetische Zwecke in Accessoires und auf Textilien eingesetzt, hatten aber kaum funktionale Wirkung. Im Bereich tragbarer Elektronik (sogenannte "Wearable Technologies"), einem Markt mit enormen Wachstumschancen, fehlen sie, da dieser Bereich von den Anwendern mehr mit Funktionalität als mit Schmuck verbunden wird. Eine der größten Herausforderungen im Bereich der "Wearable Technologies", etwa Körpersensoren, "Smart-Watches" oder Datenbrillen, stellt die Energieversorgung dar, deren abrupter Ausfall die Geräte zu oft unerwarteten Zeitpunkten funktionsunfähig macht.

Aus der Patentanmeldung US 2013/0329402 ist die Energieversorgung über eine eingebaute Solarzelle für dekorative Elemente bekannt.

Solarzellen werden gemäß der Patentschrift US 4,173,229 auch in Armbändern und Ketten eingesetzt, um einen therapeutisch wirksamen, elektrischen Strom durch den Körper der Schmuckträger zu leiten.

Das deutsche Gebrauchsmuster DE 203 03 952 U1 schlägt die Verwendung von Solarzellen in "Alarmverschlüssen" zur Sicherung von Schmuck vor.

Gegenstand der EP 2458457 A1 (CASIO) ist eine Uhr in der ein transparenter Bestandteil (2), eine Dekorplatte (7) und eine Solarzelle (5) in diese Reihenfolge in einer metallischen Kassette (1a) ausgehend von deren endseitigen Öffnung angeordnet sind, wobei die Dekorplatte lichtdurchlässig ist und einen lichtbrechenden Anteil (70) bestehend aus einem konkav-konvexen Oberflächenanteil aufweist, der sich auf der Seite der Solarzelle befindet, und eine halbdurchlässige Reflexionsplatte (6) mit sowohl lichtdurchlässigen wie lichtreflektierenden Eigenschaften zwischen der Dekorplatte und der Solarzelle, wobei die halbdurchlässige Reflexionsplatte einen metallfreien Film (61) aufweist, der aus der Dampfphase abgeschieden worden ist. Ein Querschnitt zeigt, dass die Uhr auf der Oberseite eine lichtdurchlässige Platte aufweist, die von beiden Seiten plan geschliffen ist und keine Facetten aufweist. Es folgt eine Dekorplatte mit einer konkav-konvexen Oberflächenstruktur, die an der Unterseite mit der Solarzelle verbunden ist. Bei der konkav-konvexen-Schicht handelt es sich um eine Abfolge von Bergen und Tälern, d.h. die Uhr selbst weist keine umfassend konvex gekrümmten Bereiche auf.

CN 101 606 769 A (DEFANG CHEN), im ersten Absatz von Seite 3, bezieht sich auf "eine Edelstein-Verbindungsstruktur, die zum technischen Gebiet der Maschinen und Edelsteine gehört" (gemäß einer maschinellen Übersetzung davon).

DE 44 33 623 A1 (DUONG GILBERT DR), in den Zeilen 3 bis 9 von Spalte 1, bezieht sich auf "Hilfe optischer Leuchtkörper am Schmuck, subjektive Kommunikationssignale in dynamischer Weise zu erzeugen, um damit gezielt seinen Mitmenschen Informationen zum Zwecke der Kommunikationsverbesserung zu übermitteln".

GB 2 472 805 A (NEVILL PAUL ANTHONY), im ersten Absatz von Seite 1, bezieht sich auf "eine Vorrichtung, um Schmuck selbst bei schwachen Lichtverhältnissen mit hoher Helligkeit funkeln zu lassen" (gemäß einer maschinellen Übersetzung davon).

US 5 690 412 A (SHELDON JOSEPH M), in den Zeilen 4 bis 9 von Spalte 1, bezieht sich auf "beleuchtete Displays und insbesondere auf Zier- oder Modeschmuck oder dergleichen mit in Broschen, Gürtelschnallen, Armbändern und Frisurenornamenten angebrachten Edelsteinfassungen, die von einer in sich geschlossenen Batteriequelle beleuchtet werden, die periodisch durch Sonnenenergie aufgeladen wird" (gemäß einer maschinellen Übersetzung davon).

US 2014/260424 A1 (WARREN MICHAEL K), in Absatz [0002], bezieht sich auf "ein RFID-Tag in einem Schmuckstück" (gemäß einer maschinellen Übersetzung davon).

JP 2010 151511 A (CITIZEN WATCH CO LTD), in Absatz [0001], bezieht sich auf "eine Uhr mit einer Windschutzscheibe, die mit facettenreichen Schliffen verziert wurde, insbesondere eine Uhr, die eine hochdekorative Behandlung der Windschutzscheibe auf der Oberseite des Uhrengehäuses aufweist und über eine hervorragende Solarstromerzeugungsfunktion verfügt" (gemäß einer maschinellen Übersetzung davon).

JP S61 76400 A (OKURA DENKI CO LTD), in Absatz [0001], bezieht sich auf "einen simulierten Edelstein mit einer eingebauten Lichtquelle und insbesondere einen simulierten Edelstein, der für einen Ring oder eine andere Dekoration verwendet wird und Lumineszenz aufweist" (gemäß einer maschinellen Übersetzung davon).

JP H07 74380 A (SEIKOSHA KK), in Absatz [0001], bezieht sich auf "eine farbige Solarzelle" (gemäß einer maschinellen Übersetzung davon).

US 6 197 428 B1 (ROGERS DONALD Z), in den Zeilen 6 bis 10 von Spalte 1, bezieht sich auf "dekorative Gegenstände in Form von Edelsteinen und dekorative Gegenstände in Form eines Substrats und optische Interferenzbeschichtungen, so dass zumindest ein Teil des auf den Gegenstand einfallenden Lichts mit Wellenlängen zwischen 400 Nanometer und 700 Nanometer reflektiert wird" (gemäß einer maschinellen Übersetzung davon).

Bisher fehlte jegliche technische Lösung, Solarzellen dekorativ anspruchsvoll zu gestalten, so dass sie auch als Schmucksteine verarbeitet werden können. Aufgabe der vorliegenden Erfindung war es, eine Solarzelle (photovoltaische Zelle) so zu adaptieren, dass ein hoch brillantes dekoratives Srhmurkelement erhalten wird.

### BESCHREIBUNG DER ERFINDUNG

Ein erster Gegenstand der vorliegenden Erfindung gemäß Anspruch 1 betrifft ein Schmuckelement, enthaltend
(a) einen transparenten Schmuckstein mit einer facettierter Oberfläche umfassend konvex gekrümmte Bereiche, und eine der facettierten Oberfläche gegenüberliegenden planare oder konkave Seite,
(b) eine wellenlängenselektive Schicht die auf der der facettierten Oberfläche gegenüberliegenden Seite aufgebracht ist,
(c) eine photovoltaische Zelle;
wobei die wellenlängenselektive Schicht sich zwischen dem Schmuckstein und der photovoltaischen Zelle befindet, dadurch gekennzeichnet, dass die wellenlängenselektive Schicht (3,4) das einfallende Licht in einem 50-250 nm breiten Reflexionsintervall, welches im Bereich von 380-850 nm liegt, wenigstens zu 50% reflektiert, und dass die wellenlängenselektive Schicht (3,4) außerhalb des Reflexionsintervalls eine durchschnittliche Transmission > 80% im Bereich von 400-1200 nm aufweist, gemessen bei einem Einfallswinkel der Lichtstrahlen von 0°. Die Elemente (a) bis (c) sind in einer bevorzugten Ausführungsform - und zwar in dieser Reihenfolge - mit Klebstoff verbunden. In einer weiteren bevorzugten Ausführungsform besteht das Schmuckelement aus den mit Klebstoff verbundenen Elementen (a) bis (c), ebenfalls in dieser Abfolge.

Ein weiterer Gegenstand der vorliegenden Erfindung gemäß Anspruch 13 ist die Verwendung des erfindungsgemäßen Schmuckelementes als Energiequelle, insbesondere in tragbaren elektronischen Geräten. Ebenso sind Gegenstände enthaltend ein erfindungsgemäßes Schmuckelement Gegenstand der Erfindung. Beispielweise kann das Schmuckelement vorteilhaft in sogenannte "Activity Tracker" eingebaut werden, die ebenfalls Gegenstand der Erfindung sind. Weitere Anwendungsmöglichkeiten werden nachfolgend erwähnt.

Überraschenderweise wurde gefunden, dass eine Kombination aus einem transparenten Schmuckstein mit facettierter Oberfläche umfassend konvex gekrümmte Bereiche, mit einer wellenlängenselektiven Schicht und einer photovoltaischen Zelle sich als Energiequelle für die verschiedensten Zwecke eignet. Im Sinne der Erfindung werden die Begriffe photovoltaische Zelle, photovoltaisches (PV) Element und Solarzelle synonym benutzt. Die erfindungsgemäßen Verbundkörper haben nicht nur verbesserte energieversorgende Eigenschaften, sie sind gleichzeitig Schmucksteine mit hoher Brillanz. Die Erfindung stellt somit energieliefernde, funktionale Schmucksteine bereit, die nicht nur brillant sind, sondern einen Energieertrag liefern, der höher ist als bei Solarzellen, die nur mit Flachglas bedeckt sind

Die erfindungsgemäße Kombination liefert vielfältige Einsatzmöglichkeiten im Designund Technologiebereich, sowohl als Energiequelle als auch als Schmuckstein. Nachfolgend wird der transparente Schmuckstein mit facettierter Oberfläche umfassend konvex gekrümmte Bereiche auch als "optisches Element" bezeichnet. Die Schmuckelemente sind hoch-brillant und ermöglichen somit nicht nur den Einsatz als Energiequelle, sondern auch als Dekorationselement. Unter Transparenz wird die Fähigkeit von Materie verstanden, elektromagnetische Wellen hindurchzulassen (Transmission). Ist ein Material für einfallende elektromagnetische Strahlung (Photonen) eines mehr oder weniger breiten Frequenzspektrums transparent, kann diese das Material nahezu vollständig durchdringen, wird also kaum reflektiert und kaum absorbiert. Erfindungsgemäß bevorzugt wird unter Transparenz eine Transmission von wenigstens 70 % des einfallenden Lichtes verstanden, vorzugweise mehr als 80 % und besonders bevorzugt mehr als 90 %. Unter Facettierung wird erfindungsgemäß die Gestaltung einer Oberfläche eines Schmucksteins mit Vielecken oder sogenannten Polygonen (n>3) verstanden; Facettierungen werden üblicherweise durch Schleifen eines Kristallrohlings erhalten, sind aber auch durch Pressverfahren zugänglich. Die Begriffe konvex und konkav beziehen sich auf eine gedachte einhüllende Fläche oberhalb bzw. unterhalb der Facetten und die Definitionen sind analog zu Linsen in der Optik zu verstehen. Die konvexen und konkaven Bereiche können dabei sowohl symmetrisch als auch asymmetrisch sein.

Mögliche Aufbauweisen des Schmuckelements (Verbundkörper) sind in den **Abbildungen (1a) bis (1c)** dargestellt, wobei die Bezugszeichen folgende Bedeutung haben:
(1) transparenter Schmuckstein mit facettierter Oberfläche umfassend konvex gekrümmte Bereiche;
(2) photovoltaische Zelle (Solarzelle);
(3) wellenlängenselektive Beschichtung;
(4) wellenlängenselektive Folie;
(5) Klebstoff;
(6) unterschiedlicher Aufbau bezüglich der Lage der wellenlängenselektiven Schicht;
(7) Schmuckelement.

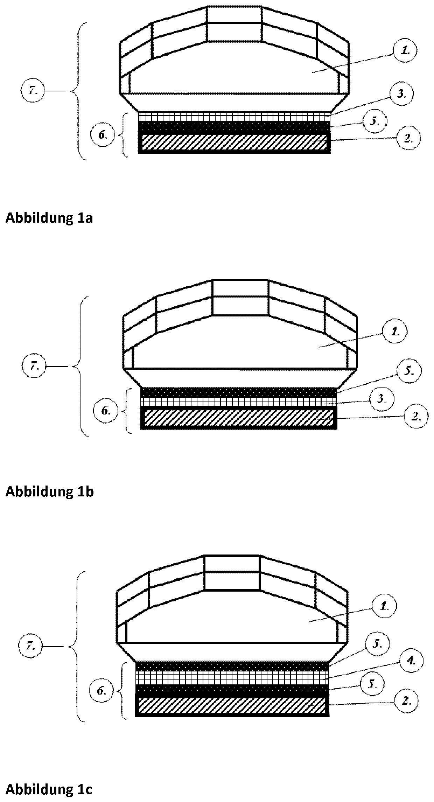

Die wellenlängenselektive Beschichtung (*vide infra*) kann sich in einer erfindungsgemäßen Ausführungsform direkt auf der der Facettierung gegenüberliegenden planaren Seite befinden, also auf der "Rückseite" des plan-konvex-konkaven oder des plan-konvexen Schmucksteins **(****Abb. 1a****),** der mit der Solarzelle verklebt ist. In einer weiteren erfindungsgemäßen Ausführungsform kann sich die wellenlängenselektive Beschichtung auf der Solarzelle befinden, die mit dem Schmuckstein (1) verklebt wird **(****Abb. 1b****).** In einer weiteren Ausführungsform **(****Abb. 1c****)** ist eine wellenlängenselektive Folie mit der Solarzelle und mit dem Schmuckstein (1) mittels zweier Klebstoffschichten verbunden **(****Abb. 1c****).** Es sei angemerkt, dass eine Verklebung der einzelnen Teile nicht zwingend notwendig ist.

Erfindungsgemäß könnte die wellenlängenselektive Schicht prinzipiell auch auf der facettierten Oberfläche aufgebracht werden; allerdings ist dies aufgrund des möglichen mechanischen Abriebs der Schicht eine der weniger bevorzugten Ausführungsformen. Die photovoltaische Zelle kann ebenso durch Abscheidung beziehungsweise Aufdampfen von Halbleitermaterialien direkt auf dem optischen Element hergestellt werden, muss also nicht notwendigerweise verklebt werden.

Das Schmuckelement bietet die Möglichkeit, verschiedene Geräte aus dem Bereich "Wearable Technologies" komplett energieautark zu betreiben oder aber deren Laufzeit abhängig vom einfallenden Licht signifikant zu erhöhen. Durch das permanente Nachladen des Energiespeichers, wird ein gewisses Energieniveau erhalten. Dies erlaubt eine Reduzierung der Ladungsträgerkapazität und folglich des Volumens des Energiespeichers. Daraus ergeben sich Vorteile für das Design des Produkts, beispielsweise eine kompaktere Bauweise, die wiederum zur Kostenreduktion des Produktes beitragen kann. Da das vollständige Laden bzw. Entladen eines konventionellen Energiespeichers durch ein externes Netzteil entfällt, resultiert eine deutlich erhöhte Lebensdauer der derzeit in den Geräten verwendeten Energiespeicher.

Eine Anwendung des Schmuckelements stellen etwa Finger- bzw. Ohrringe dar, bei denen es als Schmuckstein fungiert und gleichzeitig die nötige Energie für eine integrierte Sensorik samt Sendeeinheit liefert. Derartige Systeme können zur transkutanen, optischen Messung etwa von Laktat, Glucose oder Melatonin im Blut dienen. Das Schmuckelement erlaubt in Verbindung mit einer speziellen Dünnschichtbatterie und stark miniaturisierter Elektronik erstmals die Integration von Sensorik in ein kompaktes Schmuckstück. So kann beispielsweise ein mit dem erfindungsgemäßen Schmuckelement ausgerüsteter Ring - ohne Batteriewechsel - zur kontinuierlichen Messung bestimmter Körperfunktionen eingesetzt werden.

Auch das partielle Aufladen mobiler Geräte, wie beispielsweise von Mobiltelefonen, Laptops, GPS-Systemen oder Tablets ist durch eine Vielzahl von in Serie bzw. parallel geschalteter Schmuckelemente möglich. Bei Aufbringung einer Vielzahl solcher Schmuckelemente auf Kleidung und Accessoires, beispielsweise Taschen und Rucksäcke, können darin befindliche mobile Geräte induktiv geladen werden. Sogenannte "Energiearmbänder", welche die erfindungsgemäßen Schmuckelemente enthalten und mit Micro-USB-Steckern versehen sind, können als mobile Ladestationen verwendet werden. Das erfindungsgemäße Schmuckelement kann auch Energie für sogenannte schaltbare Effekte zur Verfügung stellen, beispielsweise für eine Farbänderung eines Schmucksteins oder beispielsweise die Displayfunktionen einer sogenannten "Smart Watch".

Das Schmuckelement oder eine Mehrzahl von Schmuckelementen kann in ein Armband integriert werden, um beispielsweise eine Smart-Watch oder einen Aktivitätssensor (activity tracker) mit Energie zu versorgen. Eine zuverlässige elektrische Verschaltung der Schmuckelemente untereinander kann erreicht werden, wenn man die Schmuckelemente über spezielle Fassungen miteinander verbindet. Eine Energieweiterleitung von den Schmuckelementen zum Produkt-Teil, das die Energie benötigt, ist beispielsweise über einen speziellen Federsteg (hauptsächlich bei Uhren) oder durch Federkontaktstifte (Pogo-Pins) möglich.

### TRANSPARENTER SCHMUCKSTEIN MIT FACETTIERTER OBERFLÄCHE UMFASSEND KONVEX GEKRÜMMTE BEREICHE

Der Schmuckstein kann aus einer Vielzahl verschiedenartiger Materialien gefertigt sein, beispielsweise aus transparentem Glas, Kunststoff, transparenter Keramik oder transparenten Edelsteinen oder Halbedelsteinen. Erfindungsgemäß bevorzugt sind facettierte, transparente Schmucksteine, die aus Glas oder aus Kunststoff hergestellt sind, da diese am kostengünstigsten sind und am leichtesten mit Facetten versehen werden können. Erfindungsgemäß besonders bevorzugt ist die Verwendung von Glas. Die Schmucksteine umfassen konvex gekrümmte oder konvex und konkav gekrümmte Bereiche. Dies bedeutet, dass auf der facettierten Seite neben den konvex gekrümmten Bereichen auch konkav gekrümmte Bereiche vorhanden sein können. Die der facettierten Seite gegenüberliegende Seite des Schmucksteins ist entweder plan (bevorzugt) oder aber konkav. Erfindungsgemäß bevorzugt sind Schmucksteine mit plan-konvexer oder plan-konvex-konkaver Geometrie, da sie die kosteneffizienteste Applikation von kristallinen Solarzellen ermöglichen. Besonders bevorzugt sind Schmucksteine mit konvexer, insbesondere plan-konvexer Geometrie.

### GLAS

Die Erfindung ist bezüglich der Zusammensetzung des Glases prinzipiell nicht limitiert, solange dieses transparent (vide supra) ist. Unter Glas wird eine eingefrorene unterkühlte Flüssigkeit verstanden, die einen amorphen Festkörper bildet. Erfindungsgemäß können sowohl oxidische Gläser als auch Chalkogenidgläser, metallische Gläser oder nicht-metallische Gläser eingesetzt werden. Auch Oxy-Nitrid-Gläser können geeignet sein. Es kann sich um Einkomponenten- (z.B. Quarzglas) oder Zweikomponenten- (z.B. Alkaliboratglas) oder Mehrkomponentengläser (Kalk-Natron-Glas) handeln. Das Glas kann durch Schmelzen, durch Sol-Gel-Prozesse oder auch durch Stoßwellen hergestellt werden. Die Verfahren sind dem Fachmann bekannt. Erfindungsgemäß bevorzugt sind anorganische Gläser, insbesondere oxidische Gläser. Hierzu gehören Silikatgläser, Boratgläser oder Phosphatgläser. Besonders bevorzugt sind bleifreie Gläser.

Für die Herstellung der facettierten transparenten Schmucksteine werden silikatische Gläser bevorzugt. Silikatische Gläser haben gemeinsam, dass ihr Netzwerk hauptsächlich aus Siliziumdioxid (SiO₂) gebildet wird. Durch Zugabe weiterer Oxide wie beispielsweise Aluminiumoxid oder verschiedener Alkalioxide entstehen die Alumo- oder Alkali-Silikatgläser. Treten als Hauptnetzwerkbildner eines Glases Phosphorpentoxid oder Bortrioxid auf, spricht man von Phosphat- bzw. Boratgläsern, deren Eigenschaften ebenfalls durch Zugabe weiterer Oxide eingestellt werden können. Auch diese Gläser sind im Sinne der Erfindung einsetzbar. Die genannten Gläser bestehen größtenteils aus Oxiden, weshalb man sie zusammenfassend als oxidische Gläser bezeichnet.

In einer erfindungsgemäß bevorzugten Ausführungsform enthält die Glaszusammensetzung folgende Komponenten:
(a) etwa 35 bis etwa 85 Gew.-% SiO₂;
(b) 0 bis etwa 20 Gew.-% K₂O;
(c) 0 bis etwa 20 Gew.-% Na₂O;
(d) 0 bis etwa 5 Gew.-% Li₂O;
(e) 0 bis etwa 13 Gew.-% ZnO;
(f) 0 bis etwa 11 Gew.-% CaO;
(g) 0 bis etwa 7 Gew.-% MgO;
(h) 0 bis etwa 10 Gew.-% BaO;
(i) 0 bis etwa 4 Gew.-% Al₂O₃;
(j) 0 bis etwa 5 Gew.-% ZrO₂;
(k) 0 bis etwa 6 Gew.-% B₂O₃;
(l) 0 bis etwa 3 Gew.-% F;
(m) 0 bis etwa 2,5 Gew.-% Cl.

Alle Mengenangaben sind dabei so zu verstehen, dass sie sich mit ggf. weiteren Komponenten zu 100 Gew.-% ergänzen. Die Facettierung der Schmucksteine wird üblicherweise durch Schleif- und Poliertechniken erhalten, die dem Fachmann hinlänglich bekannt sind.

Erfindungsgemäß geeignet ist beispielsweise ein bleifreies Glas, insbesondere das von der Firma Swarovski für die Chessboard Flat Backs verwendete Glas (Katalog-Nr. 2493), welches eine Transmission von > 95 % im Bereich 380 - 1200 nm aufweist.

### KUNSTSTOFF

Als weiterer Rohstoff für die Herstellung des facettierten transparenten Schmucksteins (a) können transparente Kunststoffe eingesetzt werden. Erfindungsgemäß geeignet sind alle Kunststoffe, die nach dem Härten der Monomere transparent sind; diese sind dem Fachmann hinlänglich bekannt. Hier finden unter anderem folgende Materialien Verwendung:
- Acrylglas (Polymethylmetacrylate, PMMA),
- Polycarbonat (PC),
- Polyvinylchlorid (PVC),
- Polystyrol (PS),
- Polyphenylenether (PPO),
- Polyethylen (PE),
- Poly(n-methylmethacrylimid (PMMI).

Die Vorteile der transparenten Kunststoffe gegenüber Glas liegen insbesondere im geringeren spezifischen Gewicht, das nur rund die Hälfte des Glases beträgt. Auch andere Materialeigenschaften sind gezielt einstellbar. Kunststoffe sind zudem oft einfacher zu bearbeiten als Glas. Nachteilig wirkt sich der im Vergleich zu Glas geringe Elastizitätsmodul und die geringe Oberflächenhärte sowie der massive Festigkeitsabfall bei Temperaturen ab ca. 70°C aus. Ein erfindungsgemäß bevorzugter Kunststoff ist Poly(n-methylmethacrylimid), der beispielsweise von Evonik unter dem Namen Pleximid^{®} TT70 vertrieben wird. Pleximid^{®} TT70 hat einen Brechungsindex von 1,54 und einen Transmissionsgrad von 91 %, gemessen nach ISO 13468-2 unter Verwendung von D65-Normlicht.

### GEOMETRIE

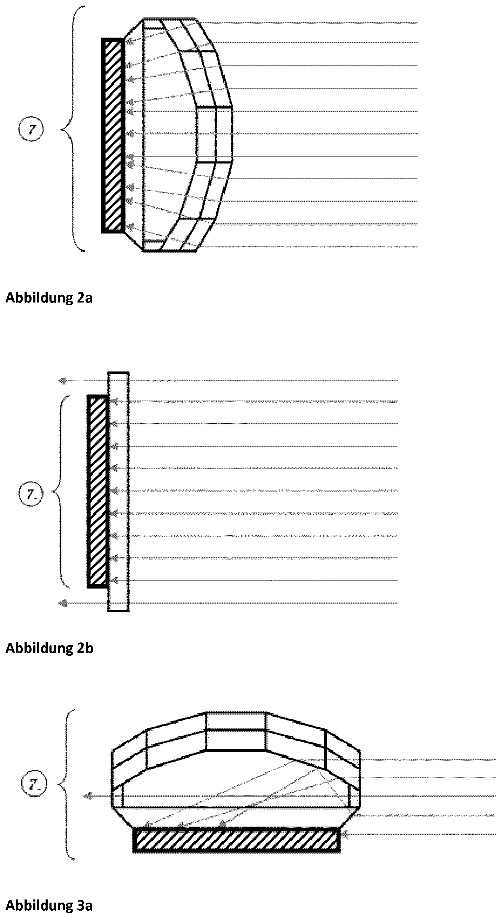

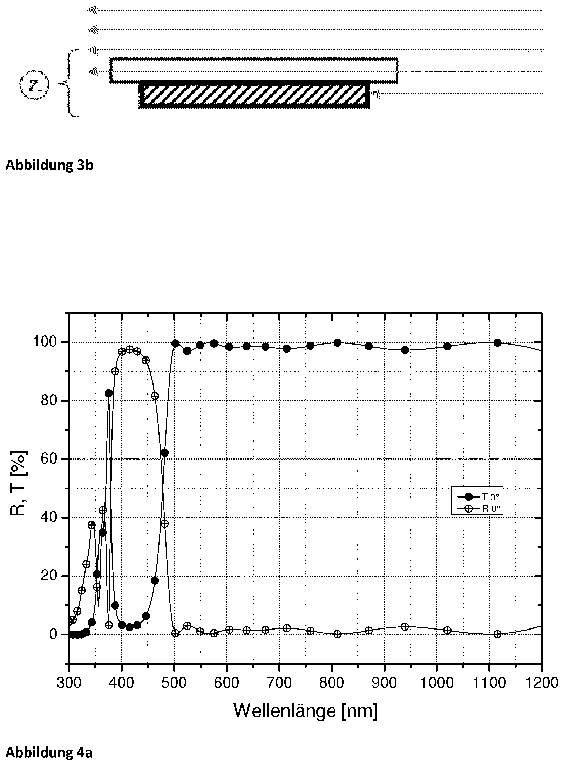

Die geometrische Ausgestaltung des facettierten, transparenten Schmucksteins ist prinzipiell nicht limitiert und hängt vorwiegend von Designaspekten ab. Der Schmuckstein ist vorzugsweise quadratisch, rechteckig oder rund. Der facettierte transparente Schmuckstein weist vorzugsweise eine konvexe, insbesondere eine plan-konvexe Geometrie auf (vgl. Abbildung 2a). Vorzugsweise enthält der Schmuckstein eine Vielzahl von Facetten auf der vorzugsweise konvex gekrümmten Seite; bevorzugt sind rechteckige, insbesondere quadratische Facetten, da diese zur Optimierung der Energieausbeute beitragen. Die Geometrie des Schmucksteins mit konvexen und ggf. zusätzlichen konkaven Bereichen erhöht die Lichtausbeute durch Vergrößerung der gesamten Oberfläche. Während die wellenlängenselektive Schicht (*vide infra*) einen negativen Effekt bezüglich der Lichtausbeute hat, weil ein bestimmter Teil des einfallenden Lichtes reflektiert wird, wird dieser Verlust durch die spezielle Geometrie mit konvexen und ggf. konkav gekrümmten Bereichen in Kombination mit den Facetten mehr als kompensiert. Insbesondere die konvexe Geometrie des Schmucksteines trägt dazu bei, dass auch die Winkelabhängigkeit der Energieausbeute der Solarzelle entscheidend reduziert wird. Gerade im Hinblick auf tragbare Elektronik, bei der eine Ausrichtung zur Lichtquelle kaum möglich ist, ist eine Reduzierung der Winkelabhängigkeit von sehr großer Bedeutung. Durch die Kombination aus Konvexität und Facettierung (Abb. 2a) werden die Lichtstrahlen auf der Oberfläche des photovoltaischen Elements gebündelt und die Energieausbeute wird im Vergleich zu einer planaren Geometrie (Abb. 2b) deutlich erhöht. Gleichzeitig wird, wie Abb. 3a zeigt, die Winkelabhängigkeit im Vergleich zu einer üblicherweise zum Verkapseln von Solarzellen verwendeten dünnen Platte (Abb. 3b) drastisch reduziert. Durch die konvexe Krümmung in Kombination mit der Facettierung und die daraus resultierende zusätzliche Fläche werden die Lichtstrahlen, die auf das Schmuckelement fallen, zum Lot auf die Solarzelle gebrochen. Durch die Facettierung kommt es zu einer Mehrfachreflexion der Lichtstrahlen (light trapping) und somit zu einer Steigerung der Lichtausbeute.

In einer erfindungsgemäß bevorzugten Ausführungsform beträgt der Oberflächenanteil des konvexen Bereiches maximal 1/3 der facettierten Gesamtoberfläche des Schmucksteines. In diesem Fall ist die Lichtausbeute einer konvex-konkaven Geometrie ähnlich der einer ausschließlich konvexen Geometrie. Dies konnten Simulationen zeigen (vide infra).

Die Art der Facettierung hängt eng mit der Geometrie des optischen Elementes zusammen. Prinzipiell ist die geometrische Form der Facetten nicht limitiert. Erfindungsgemäß bevorzugt sind quadratische oder rechteckige Facetten, insbesondere in Kombination mit einem quadratisch oder rechteckig dimensionierten transparenten Schmuckstein mit plankonvexer Geometrie. Ebenso können jedoch runde, facettierte Schmucksteine verwendet werden.

### WELLENLÄNGENSELEKTIVE SCHICHT

Die wellenlängenselektive Schicht ermöglicht, dass das Schmuckelement überhaupt als Schmuckstein verwendet werden kann. Das Schmuckelement erscheint dadurch brillant. Die wellenlängenselektive Schicht befindet sich zwischen dem transparenten, facettierten Schmuckstein umfassend konvex gekrümmte Bereiche und dem photovoltaischen Element. Sie wird erfindungsgemäß bevorzugt auf zwei verschiedene Weisen verwirklicht werden: durch eine wellenlängenselektive Folie oder aber durch eine wellenlängenselektive Beschichtung, die durch PVD, CVD oder nasschemische Verfahren hergestellt wird. Eine wellenlängenselektive Schicht kann aber ebenso durch eine mikrostrukturierte Oberfläche erhalten werden. Die Methoden zur Mikrostrukturierung sind dem Fachmann wohl bekannt.

Durch die Reflexion eines definierten Bereichs (=Filtern) des sichtbaren Spektrums gewinnt das optische Element an Brillanz und erscheint für den Betrachter in einer bestimmten Farbe. Durch die Facettierung des Schmucksteines wird die Brillanz zusätzlich unterstützt. Die wellenlängenselektive Schicht reflektiert einen Anteil des Lichtes im Bereich von 380 - 850 nm, d.h. im vorwiegend sichtbaren Bereich. Der Anteil des Lichtes, der reflektiert wird, liegt dabei in einem möglichst schmalen Bereich des sichtbaren Spektrums, in einem nicht mehr als 50-250nm breiten Intervall. Einerseits ist dieser Anteil ausreichend, um das dekorative Element hinsichtlich der Brillanz als Schmuckstein wahrzunehmen. Andererseits werden Verluste in der Energieausbeute, resultierend aus dem reflektierten Wellenlängenbereich, minimiert. Die wellenlängenselektive Schicht reflektiert das einfallende Licht in einem 50-250nm breiten Reflexionsintervall, welches im Bereich von 380 - 850 nm liegt, wenigstens zu 50%. Vorzugsweise ist das Reflexionsintervall 50 - 200 nm breit, besonders bevorzugt 50 - 150 nm. In einer weiteren bevorzugten Ausführungsform hat die wellenlängenselektive Schicht außerhalb des Reflexionsintervalls eine durchschnittliche Transmission von > 60 %, vorzugsweise > 80% im Wellenlängenbereich von 400-1200nm (vgl. Abb.4a), gemessen bei einem Einfallswinkel der Lichtstrahlen von 0°. Vorzugsweise ist die wellenlängenselektive Schicht auf der Seite des Schmucksteines aufgebracht, die der facettierten Seite gegenüberliegt; alternativ kann sie auch direkt auf das photovoltaische Element aufgebracht werden.

Die photovoltaische Zelle (Solarzelle) kann nur einen Teil des Sonnenspektrums nutzen. Die wellenlängenselektive Schicht, die als Filter wirkt, reflektiert vorzugsweise zusätzlich den Teil des Spektrums, der im IR-Bereich liegt und von der Solarzelle nicht mehr genutzt werden kann und verhindert somit eine zusätzliche Erwärmung der Solarzelle.

Üblicherweise verlieren Solarzellen 0,47% an Energieausbeute pro °C Erwärmung, so dass die korrekte Wahl der Beschichtung von hoher Bedeutung ist. Je kürzer die einfallende Wellenlänge ist desto höher ist die Energie der Photonen (E=h·ν [eV]). Bei Silizium-Solarzellen wird eine Energie von 1.1eV benötigt um ein Elektronen Loch-Paar aus dem p/n Übergang zu schlagen; die überschüssige Energie wird in Wärme umgewandelt. Wenn beispielsweise ein Photon mit 3.1eV entsprechend der Energie bei 400nm auf die Zelle eintrifft, werden 2eV in Wärmeenergie umgewandelt und führen zu einer Reduzierung der Energieausbeute. Daher ist es erfindungsgemäß besonders vorteilhaft den kurzwelligen blauen bzw. grünen Anteil zu reflektieren (Wellenlänge: 380-490nm), da hier am meisten Wärme erzeugt wird. Prinzipiell ist es durch die wellenlängenselektive Schicht möglich, Schmuckelemente mit den verschiedensten Farben zu generieren. Um die Energieausbeute zu optimieren, ist es jedoch bevorzugt, dass die wellenlängenselektive Schicht einen Anteil aus dem kurzwelligen Bereich des sichtbaren Spektrums reflektiert.

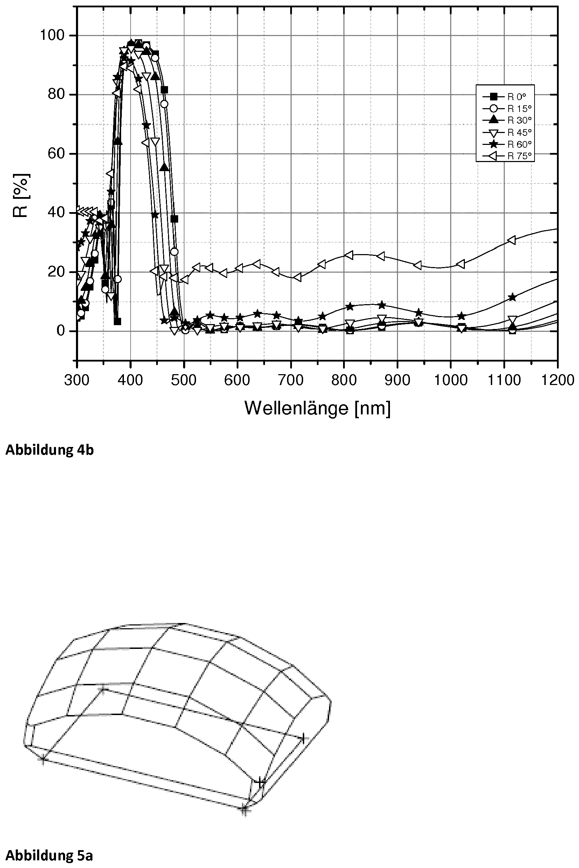

Die wellenlängenselektive Schicht zeigt eine winkelabhängige Reflexion (Abb. 4a und 4b). Das Reflexionsintervall verschiebt sich je nach Einfallswinkel des Lichtes auf die Facetten. In Abhängigkeit von der Lage der Facetten werden unterschiedliche Farbanteile reflektiert und es entsteht ein annähernd irisierender Effekt, d.h. eine graduelle Farbveränderung von Facette zu Facette, die mit einer plan-konvexen Linse ohne Facetten nicht erreicht werden kann.

Die wellenlängenselektive Schicht ist vorzugsweise zumindest partiell für UV-Licht durchlässig, um auch eine Verklebung der einzelnen Komponenten des Schmuckelementes mit UV-härtenden Klebstoffen zu ermöglichen.

### WELLENLÄNGENSELEKTIVE FOLIEN

Wellenlängenselektive Folien werden unter dem Begriff "Radiant Light Film" im Handel vertrieben. Hierbei handelt es sich um mehrschichtige polymere Filme, die auf andere Materialien appliziert werden können. Diese Lichtfolien sind Bragg-Spiegel und reflektieren einen hohen Anteil des sichtbaren Lichts und erzeugen brillante farbige Effekte. Eine reliefartige Mikrostrukturierung im Bereich von mehreren hundert Nanometern reflektiert die verschiedenen Wellenlängen des Lichtes Licht und es kommt zu Interferenzerscheinungen, wobei sich die Farben je nach Blickwinkel ändern.

Erfindungsgemäß besonders bevorzugte Folien bestehen aus mehrschichtigen polymeren Filmen, deren äußere Schicht ein Polyester ist. Solche Filme werden beispielsweise von der Firma 3M unter dem Namen Radiant Color Film CM 500 und CM 590 vertrieben. Die Filme haben ein Reflexionsintervall von 590 - 740 nm bzw. 500 - 700 nm.

Die wellenlängenselektive Folie wird vorzugsweise mittels eines Klebstoffs mit der photovoltaischen Zelle und dem facettierten transparenten Schmuckstein verbunden. Der Klebstoff sollte ebenfalls transparent sein. In einer bevorzugten Ausführungsform weicht der Brechungsindex des Klebstoffs weniger als ± 20 % von dem Brechungsindex des facettierten, transparenten Körpers mit konvexer Geometrie ab. In einer besonders bevorzugten Ausführungsform ist die Abweichung < 10 %, ganz besonders bevorzugt < 5 %. Nur auf diese Weise wird gewährleistet, dass Reflexionsverluste aufgrund der unterschiedlichen Brechungsindizes minimiert werden können. Die Brechungsindices können auch durch Aufrauhung der jeweiligen Grenzschichten aneinander angepasst werden (Mottenaugeneffekt). Sogenannte "Mottenaugenoberflächen" bestehen aus feinen Noppenstrukturen, die das Brechungsverhalten des Lichtes nicht schlagartig, sondern idealerweise kontinuierlich verändern. Dadurch werden die scharfen Grenzen zwischen den unterschiedlichen Brechzahlen beseitigt, so dass der Übergang fast fließend erfolgt und das Licht ungehindert hindurch dringen kann. Die Strukturgrößen, die dafür erforderlich sind, müssen kleiner als 300 nm sein. Durch Mottenaugeneffekte wird sichergestellt, dass die Reflexion an den Grenzschichten minimiert wird und somit eine höhere Lichtausbeute beim Durchgang durch die Grenzschichten erzielt wird.

Klebstoffe, die mittels UV ausgehärtet werden können, sind erfindungsgemäß bevorzugt. Dem Fachmann sind sowohl die UV-härtenden Klebstoffe wohl bekannt als auch die Verfahren zur Bestimmung des Brechungsindex. Erfindungsgemäß besonders bevorzugt ist die Verwendung von Acrylat-Klebstoffen, insbesondere von modifizierten Urethanacrylat-Klebstoffen. Diese werden von zahlreichen Firmen vertrieben, beispielsweise von Delo unter der Bezeichnung Delo-Photobond^{®} PB 437, ein Klebstoff, der durch UV-Licht im Bereich von 320-42 nm ausgehärtet werden kann.

### WELLENLÄNGENSELEKTIVE BESCHICHTUNG

Die Beschichtungsmaterialien sind dem Fachmann wohl bekannt. In einer bevorzugten Ausführungsform der Erfindung enthalten die wellenlängenselektiven Beschichtungen wenigstens ein Metall und/oder eine Metallverbindung, wie beispielsweise Metalloxide, Metallnitride, Metallfluoride, Metallcarbide oder eine beliebige Kombination dieser Verbindungen in beliebiger Reihenfolge, die mittels einer der gängigen Beschichtungsverfahren auf die facettierten Schmucksteine aufgebracht werden. Es können auch aufeinanderfolgende Schichten verschiedener Metalle oder Metallverbindungen aufgebracht werden. Die Verfahren zur Herstellung von Beschichtungen sowie die Beschichtungen selbst sind dem Fachmann hinlänglich bekannt. Hierzu zählen unter anderem PVD (physical vapour deposition), CVD (chemical vapour deposition), Lackieren sowie nasschemische Verfahren gemäß Stand der Technik. Erfindungsgemäß bevorzugt sind PVD-Verfahren.

Bei den PVD-Methoden handelt es sich um eine Gruppe von vakuumbasierten Beschichtungsverfahren bzw. Dünnschichttechnologien, die dem Fachmann hinlänglich bekannt sind und insbesondere zur Beschichtung von Glas und Kunststoff in der Optik- und der Schmuckindustrie eingesetzt werden. Im PVD-Prozess wird das Beschichtungsmaterial in die Gasphase überführt. Das gasförmige Material wird anschließend zum zu beschichtenden Substrat geführt, wo es kondensiert und die Zielschicht bildet. Mit einigen dieser PVD-Verfahren (Magnetronsputtern, Laserstrahlverdampfen, thermische Bedampfung, etc.) können sehr niedrige Prozesstemperaturen verwirklicht werden. Eine Vielzahl von Metallen kann auf diese Weise in sehr reiner Form in dünnen Schichten abgeschieden werden. Führt man den Prozess in Gegenwart von Reaktivgasen wie Sauerstoff durch, so lassen sich auch Metalloxide abscheiden. Ein erfindungsgemäß bevorzugtes Verfahren ist ein Beschichtungsprozess mittels Sputtern. Ein typisches Schichtsystem kann je nach Anforderung an Funktion und optische Erscheinung aus nur einer, aber auch aus einer Vielzahl von Schichten bestehen. In der Praxis beschränkt man sich meist auf Schichtanzahlen zwischen 1 und 25. Die typische Schichtdicke variiert zwischen 5 und 800 nm. Als Beschichtungsmaterialien sind erfindungsgemäß insbesondere Cr, Cr₂O₃, Ni, NiCr, Fe, Fe₂O₃, Al, Al₂O₃, Au, SiOx, Mn, Si, Si₃N₄, TiOx, Cu, Ag, Ti,CeF₃, MgF₂, Nb₂O₅, Ta₂O₅, SnO₂, ZnO₂, MgO, CeO₂, WO₃, Pr₂O₃, Y₂O₃; BaF₂, CaF₂, LaF₃, NdF₃, YF₃; ZrO₂, HfO₂, ZnS, Oxinitride von Al, Si, sowie SnZnO geeignet.

Um eine wellenlängenselektive Beschichtung zu erhalten, können beispielweise absorbierende Materialien verwendet werden, die aufgrund ihres Absorptionsverhaltens wellenlängenselektiv nur gewisse Anteile des sichtbaren Lichtes transmittieren bzw. reflektieren und dadurch farbig sind. Erfindungsgemäß bevorzugt geeignet sind Schichtsysteme, die aus dielektrischen Materialien aufgebaut sind und aufgrund von Interferenzerscheinungen nur gewisse Anteile des sichtbaren Lichtes transmittieren bzw. reflektieren und dadurch farbig wirken, beispielsweise eine Vielfach-Abfolge von TiO₂ und SiO₂. Eine erfindungsgemäß besonders bevorzugte wellenlängenselektive Beschichtung besteht aus einer alternierenden Abfolge von TiO₂ und SiO₂ in zwölf Schichten und Schichtdicken, die zwischen ca. 20-145 nm variieren. Erfindungsgemäß bevorzugt sind sogenannte Bandsperrfilter mit den Kantenlagen 380 und 480 nm, d.h. dass im Bereich 380-480 nm der größte Anteil des Lichtes reflektiert wird (=Reflexionsintervall; vgl. Abbildung 4a). Zur Herstellung von Bandsperrfiltern anderer Kantenlagen werden Schichtanzahl und Schichtdicke variiert. Für die PVD-Schichterzeugung steht eine Vielzahl handelsüblicher Maschinen zur Verfügung, beispielsweise das Modell BAK1101 der Firma Evatec.

### PHOTOVOLTAISCHES ELEMENT

Das photovoltaische Element (Solarzelle) ist ein elektrisches Bauelement, das kurzwellige Strahlungsenergie, in der Regel Sonnenlicht, direkt in elektrische Energie umwandelt. Welche Art von Solarzelle einsetzbar ist, hängt von der erforderlichen Energieversorgung sowie dem speziellen Applikationszweck ab. Für den erfindungsgemäßen Applikationszweck eignen sich insbesondere anorganische Solarzellen. Sie werden aus Halbleitermaterialien, am häufigsten aus Silicium, gefertigt. Daneben finden unter anderem auch Cadmiumtellurid, Kupfer-Indium-Gallium-Diselenid und Galliumarsenid Anwendung. Bei sogenannten Tandem-Solarzellen werden Schichten unterschiedlicher Halbleiter verwendet, beispielsweise Indiumgalliumarsenid in Kombination mit Indiumgalliumphosphid.

Neben dem Material ist die Bauweise der Solarzelle von Bedeutung. Stapeltechniken mit Materialkombinationen werden beispielsweise verwendet, um den Wirkungsgrad der Gesamtanordnung zu erhöhen. Die Materialien werden so gewählt, dass das einfallende Sonnenspektrum maximal ausgenutzt wird. Während der theoretisch erreichbare Wirkungsgrad bei ca. 43% liegt, werden in der Realität in Standard-Solarzellen nur ca. 15 bis 20 % erreicht. Verluste entstehen durch Rekombination der Ladungsträger und einhergehender Wärmeerzeugung, durch Reflexion sowie aufgrund des Serienwiderstandes. Die elektrische Spannung bei maximaler Leistung (Maximum Power Point, Leistungsanpassung) liegt bei den gebräuchlichsten Zellen (kristalline Siliziumzellen) bei etwa 0,5 V.

Die Struktur von Solarzellen wurde in den letzten Jahren optimiert, so dass möglichst viel Licht absorbiert wird und in der aktiven Schicht freie Ladungsträger erzeugt werden. Dazu wird eine Antireflexionsschicht auf die Oberseite der Solarzelle aufgetragen während die Rückseite verspiegelt wird. Die Antireflexionsschicht sorgt für die typisch bläuliche bis schwarze Farbe von Solarzellen. Die Antireflexionsschicht wird häufig aus Siliciumnitrid, Siliciumdioxid und Titandioxid hergestellt. Über die Schichtdicke der Antireflexbeschichtung wird auch die Farbe bestimmt (Interferenzfarbe). Eine gleichmäßige Schichtdicke ist wichtig, da bereits Schwankungen im Nanometer-Bereich den Reflexionsgrad erhöhen. Blaue Reflexion ergibt sich aus der Einstellung der Antireflexschicht auf den roten Teil des Spektrums - der bevorzugten Absorptionswellenlänge des Siliciums. Siliciumnitrid und Siliciumdioxid als Antireflexschicht-Materialien fungieren zusätzlich als Passivierungsschicht, die die Rekombination von Ladungsträgern an der Oberfläche herabsetzt, so dass mehr Ladungsträger zur Stromerzeugung zur Verfügung stehen. Eine weitere Effizienzsteigerung erreicht man, wenn man die frontseitigen Kontaktfinger auf der Rückseite der Solarzelle anbringt. Hierdurch wird die Abschattung auf der Frontseite vermieden, die eine geringere aktive Fläche und folglich eine geringere Lichtausbeute zur Folge hat, da bis zu 10% der Oberfläche von den Metallkontakten bedeckt ist. Rückseitige Kontaktfinger sind darüber hinaus leichter und verlustärmer elektrisch kontaktierbar als frontseitige Kontaktfinger. Erfindungsgemäß bevorzugt sind rückseitenkontaktierte Solarzellen. Diese sogenannten IBC-Zellen (Interdigitated Back Contact Cells) werden beispielsweise von der Firma SunPower vermarktet. Erfindungsgemäß geeignet sind insbesondere Solarzellen aus monokristallinem Silicium und einer Antireflexbeschichtung aus Siliciumnitrid; vorzugsweise weisen die Solarzellen eine Effizienz > 20 % auf. Erfindungsgemäß besonders geeignet ist die Sunpower^{®} C60-Solarzelle aus monokristallinem Silizium, die sich durch eine Effizienz von ca. 22.5 % auszeichnet. Die Antireflexbeschichtung aus Siliziumnitrid (Si₃N₄) hat typischerweise einen Brechungsindex von 1,9-2,5. Zur Effizienzsteigerung der Solarzellen tragen unter anderem eine Rückseitenkontaktierung, eine Rückseitenverspiegelung, eine Passivierungsschicht aus Siliciumdioxid sowie die Verwendung von n-dotiertem Silicium bei.

Die erfindungsgemäß einsetzbare Größe/Fläche der Solarzelle und des erfindungsgemäßen Schmuckelementes ist von der Anwendung und von der Bestrahlungsstärke abhängig. Bei einer Fläche von 1cm² und einer Zelleneffizienz von ca. 20% lassen sich bei einer Bestrahlungsstärke von 100 mW/cm² in direktem Sonnenlicht innerhalb einer Stunde theoretisch bis zu 20 mWh an Energie sammeln. In der Praxis wird dieser Wert aufgrund elektrischer Verluste beim Laden des Energiespeichers - sowie der Tatsache, dass eine durchschnittliche Bestrahlungsstärke von ca. 100 mW/cm² bzw. 1000 W/m² in Mitteleuropa nicht häufig erreicht wird - etwas niedriger ausfallen. Bezogen auf einen am Markt erhältlichen "ActivityTracker", der eine durchschnittliche Entladung von ca. 3 mWh/Tag aufweist, würde bei einer Fläche von 1 cm² Solarzelle eine Bestrahlungsdauer von einer Stunde pro Woche in direktem Sonnenlicht ausreichen. Aufgrund der guten Performance der IBC-Solarzellen auch unter nicht-optimalen Lichtbedingungen ist auch eine Verwendung in Innenräumen ausreichend, um einer Entladung der tragbaren elektronischen Geräte entgegenzuwirken. Im Vergleich zu direktem Sonnenlicht im Freien, ist die Bestrahlungsstärke in Räumen um den Faktor 100-200 geringer. Die anfangs genannten Sensoren zur Überwachung von Körperfunktionen zeigen eine durchschnittliche Entladung von ca. 1 bis 5 mWh/Tag. Auch hier ist eine Energieversorgung über das erfindungsgemäße Schmuckelement möglich, beispielsweise durch Integration des Schmuckelementes oder einer Vielzahl solcher Elemente in Schmuck-Designs.

In einer bevorzugten Ausführungsform der Erfindung ist das photovoltaische Element mit elektrischen Kontakten versehen, um die generierten elektrischen Ladungsträger in Form von Strom abzuleiten. Dabei werden die rückseitigen elektrischen Kontakte der Solarzelle über eine Leiterplatte kontaktiert und zu einem positiven und einem negativen Kontakt zusammengeführt.

Die Erfindung wird nachfolgend an Hand von Beispielen und Abbildungen näher illustriert ohne darauf beschränkt zu sein. Die Abbildungen zeigen folgende Gegenstände:
- **Abb. 1a:**: Aufbau eines Schmuckelements mit wellenlängenselektiver Beschichtung auf der der Facettierung gegenüberliegenden planen Seite.
- **Abb. 1b:**: Aufbau eines Schmuckelements mit wellenlängenselektiver Beschichtung auf der Solarzelle.
- **Abb. 1c:**: Aufbau eines Schmuckelements mit wellenlängenselektiver Folie.
- **Abb. 2a:**: Bündelung von Lichtstrahlen auf der Solarzelle bei plankonvexem optischem Element mit Facettierung
- **Abb. 2b:**: Strahlengang bei planarer Abdeckung der Solarzelle
- **Abb. 3a:**: Brechung von seitlich einfallenden Lichtstrahlen bei plankonvexem optischem Element mit Facettierung
- **Abb. 3b:**: Strahlengang bei seitlich einfallenden Lichtstrahlen bei planarer Abdeckung der Solarzelle
- **Abb. 4a:**: Spektrum der wellenlängenselektiven Filter-Beschichtung gemäß Tabelle 1; T = Transmission; R = Reflexion
- **Abb. 4b:**: Winkelabhängigkeit der Reflexion der wellenlängenselektiven Filter-Beschichtung R = Reflexion
- **Abb. 5a:**: Geometrie des optischen Elements; perspektivisch
- **Abb. 5b:**: Grundfläche des optischen Elements; 45º Phase an der Grundfläche
- **Abb. 6:**: Messaufbau - schematisch
- **Abb. 7a:**: Relative Änderung der Leistung im optimalen Arbeitspunkt in Abhängigkeit vom Einfallswinkel der Strahlung
- **Abb. 7b:**: Relative Änderung der Leistung im optimalen Arbeitspunkt nach Applikation optischer Elemente gemittelt über die Einfallswinkel 0-75°
- **Abb. 8:**: Geometrie des optischen Elementes für die Simulation
- **Abb. 9:**: Geometrie des optischen Elementes mit plan-konvex-konkaver Krümmung
- **Abb. 10:**: Geometrie des optischen Elementes mit plan-konkaver Krümmung (nicht Teil der Erfindung)

### GEWERBLICHE ANWENDBARKEIT

Weitere Gegenstände der Erfindung betreffen zum einen die Verwendung des Schmuckelements gemäß vorliegender Erfindung als Energiequelle, insbesondere in tragbaren elektronischen Geräten, sowie Gegenstände, insbesondere Schmuckteile wie etwa Ringe, Ketten, Armbänder und dergleichen, enthaltend wenigstens ein Schmuckelement gemäß vorliegender Erfindung.

### BEISPIELE

### MATERIALIEN

Es wurden verschiedene Schmuckelemente untersucht, die sich bezüglich Material und Geometrie unterscheiden. Die Schmuckelemente wurden aus Solarzellen und optischen Elementen aufgebaut. Die erfindungsgemäßen Beispiele wurden zusätzlich mit einer wellenlängenselektiven Schicht versehen.

**Solarzellen.** Es wurden Solarzellen des Typs Sunpower C60 (10 mm x 10 mm) verwendet.

**Optische Elemente** - **mit und ohne Beschichtung.** Die optischen Elemente aus Glas wurden durch die dem Fachmann bekannten Verfahren aus käuflich erhältlichen "Chessboard Flat Back" 2493-Elementen (30 mm x 30 mm) der Firma Swarovski gefertigt.

Die optischen Elemente aus Pleximid^{®} TT70 wurden mittels Kunststoffspritzgussverfahren in einer dafür vorgefertigten Form hergestellt. Hierfür wurde eine Spritzgießmaschine der Firma Engel vom Typ e-victory 80/50 verwendet; Temperatur Massezylinder: 210°C steigend bis 280°C, Düse 280°C; Temperatur Werkzeug: 180° DS, 140° AS; Spritzdruckgrenze: 1200 bar; Spritzgeschwindigkeit: ca. 15 cm³/sec; Prägedruck: ca. 800 bar; keine Lösungsmittel.

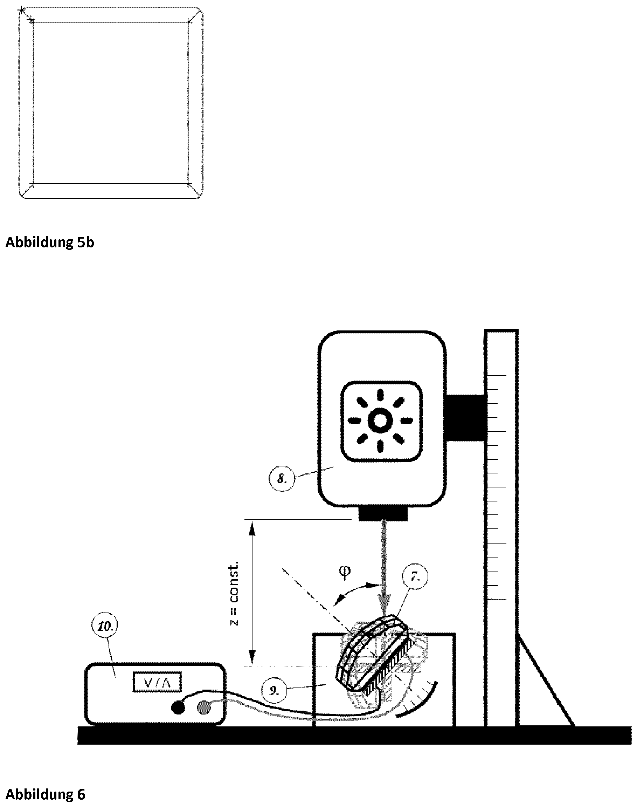

**Geometrie.** Die optischen Elemente gemäß der erfindungsgemäßen Bespiele 1 und 2 sowie der Vergleichsbespiele V2 und V3 sind facettierte Körper mit 12mm Kantenlänge und quadratischer Grundfläche mit leicht abgerundeten Ecken (Abb. 5a und 5b). An der Grundfläche ist eine Abschrägung im Winkel von 45º (Phase) angebracht, so dass die tatsächlich verbleibende Grundfläche 10mm x 10mm beträgt (vgl. Abb. 5a und 5b). Der facettierte obere Teil mit 25 Facetten in quadratischer Anordnung bildet ein Kugelsegment. Die Gesamthöhe des Körpers beträgt 5,56mm, die Eckkantenhöhe beträgt 1,93mm.

### BESCHREIBUNG DER BEISPIELE UND VERGLEICHSBEISPIELE

VERGLEICHSBEISPIELV1: 12mm x 12mm Glasplatte der Stärke 0,5 mm; Brechungsindex n = 1,52

VERGLEICHSBEISPIEL V2: Ein aus Glas gefertigtes optisches Element gemäß Abb. 5a und 5b; Dimensionen wie obenstehend beschrieben (Geometrie); ohne wellenlängenselektive Beschichtung

BEISPIEL 1: Ein aus Glas gefertigtes optisches Element gemäß Abb. 5a und 5b; Dimensionen wie obenstehend beschrieben (Geometrie); mit der nachfolgend beschriebenen wellenlängenselektiven Beschichtung

VERGLEICHSBEISPIEL V3: Ein aus Pleximid^{®} TT70 gefertigtes optisches Element gemäß Abb. 5a und 5b mit n = 1,54; Dimensionen wie obenstehend beschrieben (Geometrie); ohne wellenlängenselektive Beschichtung (Vergleichsbeispiel)

BEISPIEL 2: Ein aus Pleximid^{®} TT70 gefertigtes optisches Element laut Abb. 5a und 5b; Dimensionen wie obenstehend beschrieben (Geometrie); mit der nachfolgend beschriebenen wellenlängenselektiven Beschichtung

### WELLENLÄNGENSELEKTIVE SCHICHT

Die optischen Elemente gemäß Bespiele 1 und 2wurden in einer PVD-Anlage (*vide supra*) beschichtet. Der Aufbau der wellenlängenselektiven Beschichtung ist in **Tabelle 1** wiedergegeben:

**Tabelle 1**

| Schichtaufbau der wellenlängenselektiven Beschichtung | | |
|---|---|---|
| **N** | **Material** | **Physikalische Schichtdicke [nm]** |
| 1 | TiO₂ | 23,9 |
| 2 | SiO₂ | 43,2 |
| 3 | TiO₂ | 64,8 |
| 4 | SiO₂ | 28,7 |
| 5 | TiO₂ | 61,5 |
| 6 | SiO₂ | 33,7 |
| 7 | TiO₂ | 57,7 |
| 8 | SiO₂ | 37,5 |
| 9 | TiO₂ | 66,1 |
| 10 | SiO₂ | 30,5 |
| 11 | TiO₂ | 42,6 |
| 12 | SiO₂ | 141,4 |

### MESSAUFBAU UND MESSUNGEN

Ziel der Messungen war es, den Einfluss des optischen Elementes und der Beschichtung auf die Energieausbeute der Solarzelle in Abhängigkeit vom Einfallswinkel der Lichtstrahlen zu untersuchen.

**Schmuckelemente:** Es wurden fünf verschiedene Schmuckelemente untersucht, die aus den optischen Elementen gemäß den Beispielen und Vergleichsbeispielen und den Solarzellen des Typs Sunpower C60 (10 mm x 10 mm) aufgebaut waren.

Die Messungen wurden mit einem Oriel Instruments LED-Sonnensimulator Verasol-2 (Klasse AAA) unter Verwendung eines Keithley 2602A Sourcemeters und einer Linos Drehfassung inklusive entsprechender Halterung durchgeführt.

Eine schematische Darstellung des Messaufbaus zeigt **Abbildung 6****.** Die Bezugszeichen stehen hierbei für:
(7) Schmuckelement;
(8) Sonnensimulator;
(9) Drehfassung;
(10) Sourcemeter.

Mit Hilfe eines nach Klasse AAA (spektrale Übereinstimmung, räumliche Gleichförmigkeit, Zeitstabilität) zertifizierten Sonnensimulators (8) wurde für die komplette Versuchsreihe eine konstante Bestrahlungsstärke von 1000W/m² gewählt. Der Einfallswinkel der Strahlung der Lichtquelle auf die zu vermessenden Proben gemäß der Bespiele 1 und 2 sowie der Vergleichsbespiele V1 bis V3 wurde dabei mit Hilfe einer Drehfassung (9) variiert, auf der die Proben positioniert wurden. Der Abstand z zwischen dem Mittelpunkt der Solarzelle und dem Sonnensimulator wurde konstant gehalten (vgl. **Abb. 6****).**

Mittels Sourcemeter (10) wurde jeweils die Strom-Spannungskennlinie der Solarzellen bei einer Bestrahlungsstärke von 1000W/m² gemessen und daraus die Leistung im optimalen Arbeitspunkt (Maximum Power Point) ermittelt.

Jede der fünf Solarzellen wurde zuerst ohne optische Elemente vermessen. Der Einfallswinkel der Lichtstrahlen wurde dabei in 15°-Schritten von 0° bis 75° variiert (vgl. Abb. 6). Anschließend wurde auf jede Solarzelle jeweils ein optisches Element (*vide supra*) mit wellenlängenselektiver Beschichtung (Tabelle 1) mittels eines UV-Klebstoffs mit einem Brechungsindex n=1,461 appliziert und die komplette Messreihe wiederholt. Jede Einzelmessung wurde dreimal wiederholt; daraus wurde der arithmetische Mittelwert gebildet und die relative Standardabweichung (Standardabweichung/ Mittelwert) berechnet; die Ergebnisse sind in **Tabelle 2** zusammengefasst.

**Tabelle 2**

| Relative Änderung der Leistung der Solarzelle im optimalen Arbeitspunkt (Maximum Power Point) in Abhängigkeit vom Einfallswinkel der Lichtstrahlen für die oben beschriebenen Versuchsanordnungen | | | | | |
|---|---|---|---|---|---|
| **Einfallswinkel** | **V1** | **V2** | **1** | **V3** | **2** |
| 0° | -11,5% | 38,2% | 12,7% | 9,4% | -5,1% |
| 15° | -12,8% | 50,3% | 45,5% | 16,0% | -5,0% |
| 30° | -0,6% | 56,4% | 42,5% | 19,4% | 10,9% |
| 45° | 0,1% | 79,9% | 27,2% | 15,5% | 9,4% |
| 60° | 4,7% | 84,0% | 43,3% | 48,8% | 47,4% |
| 75° | -18,5% | 140,0% | 99,5% | 76,1% | 68,1% |
| Mittelwert 0-75° | -6,4% | 60,8% | 35,9% | 19,7% | 8,7% |

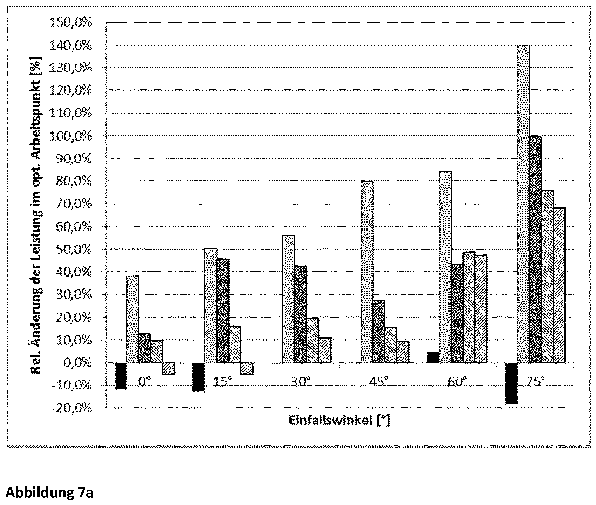

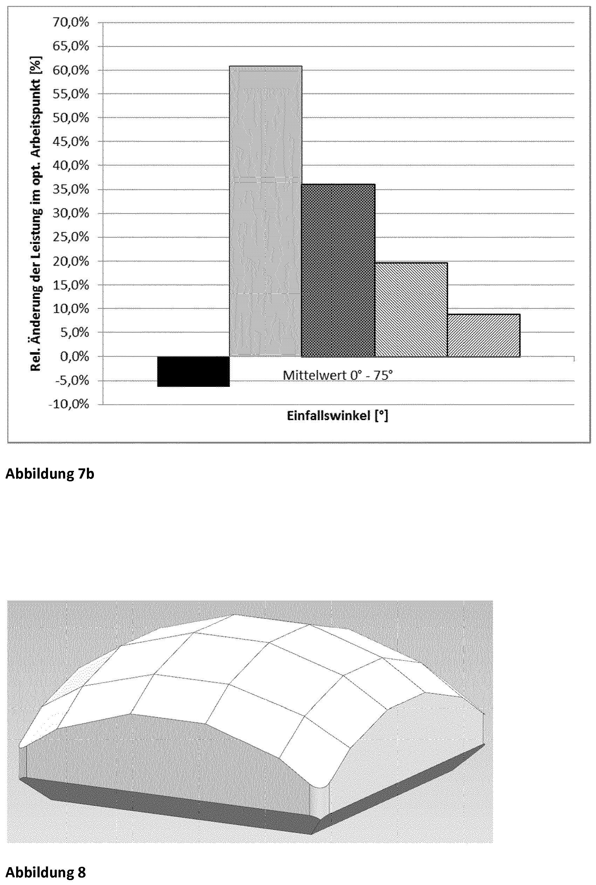

Eine graphische Auswertung der in Tabelle 2 erhaltenen Ergebnisse ist in den Abbildungen 7a und 7b zu finden. Hierbei bedeutet 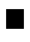 Schwarz: V1; 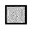 Grau: V2; 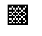 Doppelschraffur: 1; 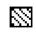 Schraffur von links oben nach rechts unten: V3; 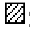 Schraffur von links unten nach rechts oben: 2;

### DISKUSSION DER ERGEBNISSE

Die Leistungsverluste bei Beispiel V1 resultieren einerseits aus Verlusten aufgrund unterschiedlicher Brechungsindizes beim Übergang Glas/UV-Klebstoff und UV-Klebstoff/Solarzelle. Andererseits geht ein Teil der Lichtstrahlen beim Auftreffen auf die Glasplatte durch Reflexion verloren. Grundsätzlich treten beide Arten des Leistungsverlusts bei allen optischen Elementen auf, jedoch sind sie bei planaren Glasplatten am größten.

Die Steigerung der Leistung der Solarzelle im optimalen Arbeitspunkt (Maximum Power Point) ist vorwiegend durch geometrische Effekte bedingt, wie in Abb. 2a/2b und Abb. 3a/3b schematisch dargestellt. Vor allem bei zunehmendem Einfallswinkel der Lichtstrahlen lässt sich die Relevanz der konvexen Geometrie mit Facettierung erkennen. Die Leistung des Schmuckelementes steigt stark an (Beispiele V2 und V3). Für Glas fällt diese noch deutlicher aus als für die aus Pleximid^{®} TT70 gefertigten optischen Elemente.

Durch die wellenlängenselektive Beschichtung (Beispiel 1 und 2) ergeben sich erwartungsgemäß Verluste beim Energieertrag im Vergleich zu V2 und V3 aufgrund der Reflexion eines Teils des sichtbaren Spektrums. Jedoch können diese, wie aus Tabelle 2 ersichtlich, durch die konvexe Geometrie in Kombination mit der Facettierung mehr als kompensiert werden.

Die Unterschiede zwischen den optischen Elementen aus Glas und Pleximid^{®} TT70 resultieren aus dem deutlich besseren Transmissionsverhalten von Glas sowie der herstellungsbedingten besseren Oberflächenqualität der Glasproben.

### COMPUTERSIMULATION

Mittels Computersimulation wurde der Einfluss einer plan-konkav-konvexen Geometrie bzw. einer plan-konkaven Geometrie des Schmucksteines auf die Leistung einer Solarzelle untersucht. Die Simulationen wurden mittels physikalischem Raytracing (Lichtstrahlverfolgung) mit dem Programm Speos der Firma Optis durchgeführt.

### Computermodell

Als Schmucksteinmodell wurden die CAD-Daten des entsprechenden Schmucksteines aus den Messungen verwendet *(vide supra).* Die Schmucksteinoberfläche wurde ideal (ohne Rauheit, d.h. ohne Oberflächendefekte) angenommen. Für die Simulation wurde der Brechungsindex des Glases des experimentellen Beispiels 1 zugrunde gelegt, der 1,56 bei λ = 550 nm beträgt.

Eine wellenlängenselektive Schicht bzw. generell eine Grenzschicht zwischen Schmuckstein und Solarzelle wurde aus Gründen der Komplexität nicht berücksichtigt. Mit den Simulationen wird lediglich der Einfluss der unterschiedlichen Geometrien (konvex, konkav) des Schmucksteines auf die Lichtausbeute untersucht. Die Einbeziehung der wellenlängenselektiven Schicht bzw. Grenzschicht ist dafür irrelevant.

Die Solarzelle wurde mit einer spiegelnden Oberfläche mit einem Reflexionsgrad von 1,3% simuliert, der vom Einfallswinkel des Lichtes unabhängig ist. Die Absorption der Solarzelle betrug 98,7%.

Die Bestrahlung des Schmucksteines mit Licht erfolgte in der Simulation analog zur Messung zentrisch von oben. Die Ausdehnung der Lichtquelle betrug 30mm x 30mm. Der Abstand der Lichtquelle vom Mittelpunkt der Auflagefläche des Schmucksteines betrug 15 mm. Der Öffnungswinkel der Lichtquelle war 2x8°. Die Lichtverteilung wurde als gaußförmig angenommen. Die Lichtquelle hatte eine Strahlungsleistung von 1 W. Als Lichtquelle wurde die Normlichtquelle D65 des Programms Speos verwendet. In Abhängigkeit vom Einfallswinkel des Lichtes *(vide infra)* trifft nur ein Teil des Lichtes auf den Schmuckstein.

### Simulationen und Ergebnisse

SIMULATION S1: Schmuckstein mit plan-konvexer Geometrie, Abb. 8. Dieser Schmuckstein entspricht dem Schmuckstein aus der Messung (Abb. 5a).

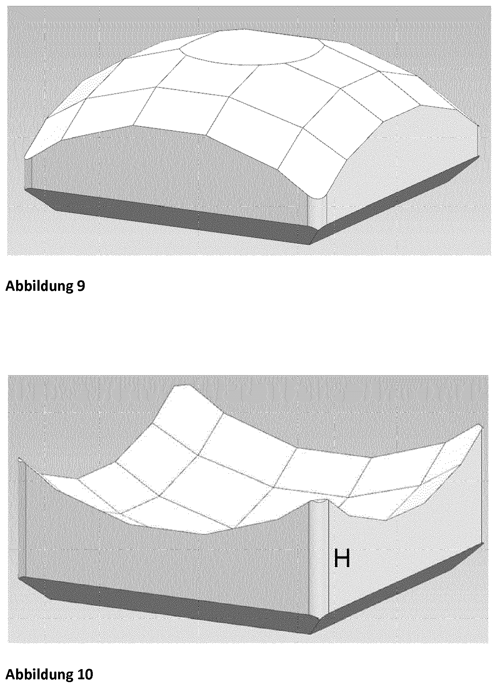

SIMULATION S2: Schmuckstein mit plan-konvex-konkaver Geometrie, Abb. 9. Die konkave Ausnehmung ist sphärisch. Man erhält sie durch eine Kugel mit einem Durchmesser von 18 mm. Der Mittelpunkt der Kugel liegt auf der Flächennormale, die durch den Mittelpunkt der Schmucksteinauflagefläche verläuft. Die konkave Ausnehmung entspricht dem Kugelsegment mit einer Höhe von 0,558 mm.

SIMULATION S3: Schmuckstein mit plan-konkaver Geometrie, Abb. 10. Die konkave Krümmung des Schmucksteines entspricht der konvexen Krümmung des ursprünglichen Schmucksteines, Abb. 8, invertiert. Die Höhe H des Schmucksteines (Abb. 10) an den Kanten beträgt 5 mm.

Ziel der Simulationen war es, den Einfluss der Geometrie (konkav, konvex) des optischen Elementes (Schmucksteines) auf das Absorptionsverhalten und somit auf die Energieausbeute der Solarzelle in Abhängigkeit vom Einfallswinkel der Lichtstrahlen zu untersuchen.

Der Einfallswinkel der Lichtstrahlen wurde in der Simulation analog zur Messung *(vide supra)* variiert. Berechnet wurde die absorbierte Strahlungsleistung in Watt der modellierten Solarzelle. Die relative Abweichung der absorbierten Strahlungsleistung in Prozent ergibt sich gemäß 100 x (S2 - S1)/S1 bzw. 100 x (S3-S1)/S1 und wurde bei verschiedenen Einfallswinkeln bestimmt (Tabelle 3).

**Tabelle 3**

| Absorbierte Leistung der Solarzelle in Abhängigkeit vom Einfallswinkel der Lichtstrahlen für die oben bieschriebenen Simulationsmodelle, und die relative Abweichung der Simulationswerte 100 x (S2 - S1)/S1 bzw. 100 x (S3-S1)/S1. | | | | | |
|---|---|---|---|---|---|
| **Einfallswinkel** | **S1 [mW]** | **S2 [mW]** | **Abweichung in %: 100 x (S2 - S1)/S1** | **S3 [mW]** | **Abweichung in %: 100 x (S3-S1)/S1** |
| 0° | 598,2 | 598.3 | 0,017 | 414.9 | -30,642 |
| 15° | 584,7 | 584.0 | -0,12 | 433.3 | -25,894 |
| 30° | 526,8 | 527.2 | 0,076 | 451.1 | -14,37 |
| 45° | 454,6 | 449.9 | -1,034 | 412.2 | -9,327 |
| 60° | 364.6 | 353.7 | -2,99 | 320.8 | -12,013 |
| 75° | 219.8 | 214.3 | -2,502 | 147.7 | -32,803 |

### DISKUSSION DER ERGEBNISSE

Die relative Abweichung der Werte von S3 in Bezug auf S1 (Spalte 6), Tab. 3, zeigt, dass bei einer rein konkaven Geometrie die absorbierte Strahlungsleistung stark abnimmt. Das ist zu erwarten, da die konkave Geometrie einen streuenden Effekt hat.

Ist der Oberflächenanteil der konkaven Krümmung hingegen maximal 1/3 (Abb. 9), der gekrümmten Oberfläche (vgl. Simulation 2; Spalte 4 in Tabelle 3), dann ist die Abweichung der Leistungswerte vernachlässigbar klein.

Die relativen Abweichungen (vgl. Tabelle 3, Spalten 4 und 6) nehmen in Bezug auf den Einfallswinkel nicht kontinuierlich ab. Das ist darauf zurückzuführen, dass auch auf die nicht-gekrümmten Seitenflächen des Schmucksteines Lichtstrahlen treffen und daher innerhalb des Schmucksteines zusätzliche Reflexionen auftreten können.

Die Simulationsergebnisse zeigen, dass der Einfluss einer konkaven Krümmung, die einen Oberflächenanteil bis zu maximal 1/3 der gekrümmten Schmucksteinoberfläche hat, bezüglich der Effizienz einer Solarzelle vernachlässigbar ist.

## Patentansprüche

1. Schmuckelement (7), enthaltend
(a) einen transparenten Schmuckstein (1) mit einer facettierten Oberfläche umfassend konvex gekrümmte Bereiche, und eine der facettierten Oberfläche gegenüberliegende planare oder konkave Seite,
(b) eine wellenlängenselektive Schicht (3,4) die auf der der facettierten Oberfläche gegenüberliegenden Seite aufgebracht ist, und
(c) eine photovoltaische Zelle (2);
wobei die wellenlängenselektive Schicht (3,4) sich zwischen dem Schmuckstein (1) und der photovoltaischen Zelle (2) befindet,
**dadurch gekennzeichnet, dass** die wellenlängenselektive Schicht (3,4) das einfallende Licht in einem 50-250 nm breiten Reflexionsintervall, welches im Bereich von 380-850 nm liegt, wenigstens zu 50% reflektiert, und dass die wellenlängenselektive Schicht (3,4) außerhalb des Reflexionsintervalls eine durchschnittliche Transmission > 80% im Bereich von 400-1200 nm aufweist, gemessen bei einem Einfallswinkel der Lichtstrahlen von 0°.

2. Schmuckelement (7) nach Anspruch 1, **dadurch gekennzeichnet, dass** der Schmuckstein (1) aus Glas oder aus Kunststoff gefertigt ist

3. Schmuckelement (7) nach wenigstens einem der vorhergehenden Ansprüche, **dadurch gekennzeichnet, dass** der Schmuckstein (1) eine plan-konvexe Geometrie oder eine plan-konvex-konkave Geometrie aufweist.

4. Schmuckelement (7) nach wenigstens einem der vorhergehenden Ansprüche, **dadurch gekennzeichnet, dass** die wellenlängenselektive Schicht (3,4) ausgewählt ist aus einer wellenlängenselektiven Beschichtung (3) oder einer wellenlängenselektive Folie (4).

5. Schmuckelement (7) nach Anspruch 4, **dadurch gekennzeichnet, dass** die wellenlängenselektive Beschichtung (3,4) wenigstens ein Metall und/oder eine Metallverbindungen enthält.

6. Schmuckelement (7) nach Anspruch 4 oder Anspruch 5, **dadurch gekennzeichnet, dass** die wellenlängenselektive Beschichtung (3,4) wenigstens eine Verbindung umfasst, die ausgewählt ist aus der Gruppe, die gebildet wird von Cr, Cr₂O₃, Ni, NiCr, Fe, Fe₂O₃, Al, Al₂O₃, Au, SiOₓ, Mn, Si, Si₃N₄, TiOₓ, Cu, Ag, Ti,CeF₃, MgF₂, Nb₂O₅, Ta₂O₅, SnO₂, ZnO₂, MgO, CeO₂, WO₃, Pr₂O₃, Y₂O₃; BaF₂, CaF₂, LaF₃, NdF₃, YF₃; ZrO₂, HfO₂, ZnS, Oxinitride von Al, Si, und SnZnO oder eine beliebige Kombination dieser Verbindungen in beliebiger Schichtabfolge.

7. Schmuckelement (7) nach wenigstens einem der vorhergehenden Ansprüche 4 bis 6, **dadurch gekennzeichnet, dass** die wellenlängenselektive Beschichtung (3,4):
(a) eine Vielfach-Abfolge von TiO₂ und SiO₂ Schichten aufweist; oder
(b) aus einer alternierenden Abfolge von TiO₂ und SiO₂ in zwölf Schichten und Schichtdicken besteht, die zwischen ca. 20-145 nm variieren.

8. Schmuckelement (7) nach wenigstens einem der vorhergehenden Ansprüche 1 bis 4, **dadurch gekennzeichnet, dass** die wellenlängenselektive Schicht (3,4) eine wellenlängenselektive Folie ist, die auf der der facettierten Seite gegenüberliegenden Seite des Schmucksteins (1) und auf der photovoltaischen Zelle (2) aufgebracht ist.

9. Schmuckelement (7) nach wenigstens einem der vorhergehenden Ansprüche, **dadurch gekennzeichnet, dass** die wellenlängenselektive Schicht (3,4) einen Anteil des Lichtes im Bereich von 380 - 850 nm reflektiert.

10. Schmuckelement (7) nach wenigstens einem der vorhergehenden Ansprüche, **dadurch gekennzeichnet, dass** die photovoltaische Zelle (2) eine rückseitenkontaktierte Solarzelle ist.

11. Schmuckelement (7) nach wenigstens einem der vorhergehenden Ansprüche, **dadurch gekennzeichnet, dass**:
(i) die Komponenten (a), (b) und (c) des Schmuckelements (7) mittels eines Klebstoffs (5) verbunden sind; oder
(ii) die Komponenten (a), (b) und (c) des Schmuckelements (7) mittels eines Klebstoffs (5) verbunden sind, und wobei der Brechungsindex des Klebstoffs (5) weniger als ± 20 % von dem Brechungsindex des Schmucksteins (1) abweicht.

12. Tragbares elektronisches Gerät enthaltend wenigstens ein Schmuckelement (7) gemäß wenigstens einem der vorhergehenden Ansprüche 1 bis 11.

13. Verwendung des Schmuckelements (7) gemäß wenigstens einem der vorhergehenden Ansprüche 1 bis 11 als Energiequelle, insbesondere in tragbaren elektronischen Geräten.

## Claims

1. Ornamental element (7) containing
(a) a transparent gemstone (1) having a faceted surface comprising convexly curved regions, and a planar or concave face opposite the faceted surface,
(b) a wavelength-selective layer (3, 4) which is applied to the face opposite the faceted surface, and
(c) a photovoltaic cell (2);
the wavelength-selective layer (3, 4) being located between the gemstone (1) and the photovoltaic cell (2), **characterised in that** the wavelength-selective layer (3, 4) reflects at least 50% of the incident light in a 50-250 nm wide reflection interval, which is in the range of 380-850 nm, and that the wavelength-selective layer (3, 4) outside the reflection interval has an average transmission > 80% in the range of 400-1200 nm, measured at an angle of incidence of the light rays of 0°.

2. Ornamental element (7) according to claim 1, **characterised in that** the gemstone (1) is made of glass or plastics material.

3. Ornamental element (7) according to at least one of the preceding claims, **characterised in that** the gemstone (1) has a plano-convex geometry or a plano-convex-concave geometry.

4. Ornamental element (7) according to at least one of the preceding claims, **characterised in that** the wavelength-selective layer (3, 4) is selected from a wavelength-selective coating (3) or a wavelength-selective film (4).

5. Ornamental element (7) according to claim 4, **characterised in that** the wavelength-selective coating (3, 4) contains at least one metal and/or a metal compound.

6. Ornamental element (7) according to either claim 4 or claim 5, **characterised in that** the wavelength-selective coating (3, 4) comprises at least one compound which is selected from the group formed by Cr, Cr₂O₃, Ni, NiCr, Fe, Fe₂O₃, Al, Al₂O₃, Au, SiOₓ, Mn, Si, Si₃N₄, TiOₓ, Cu, Ag, Ti, CeF₃, MgF₂, Nb₂O₅, Ta₂O₅, SnO₂, ZnO₂, MgO, CeO₂, WO₃, Pr₂O₃, Y₂O₃; BaF₂, CaF₂, LaF₃, NdF₃, YF₃; ZrO₂, HfO₂, ZnS, oxynitrides of Al, Si, and SnZnO or any combination of these compounds in any layer sequence.

7. Ornamental element (7) according to at least one of the preceding claims 4 to 6, **characterised in that** the wavelength-selective coating (3, 4):
(a) has a multiple sequence of TiO₂ and SiO₂ layers; or
(b) consists of an alternating sequence of TiO₂ and SiO₂ in twelve layers and layer thicknesses that vary between approx. 20-145 nm.

8. Ornamental element (7) according to at least one of the preceding claims 1 to 4, **characterised in that** the wavelength-selective layer (3, 4) is a wavelength-selective film which is applied to the face of the gemstone (1) opposite the faceted face and is applied to the photovoltaic cell (2).

9. Ornamental element (7) according to at least one of the preceding claims, **characterised in that** the wavelength-selective layer (3, 4) reflects a portion of the light in the range of 380-850 nm.

10. Ornamental element (7) according to at least one of the preceding claims, **characterised in that** the photovoltaic cell (2) is a rear-contact solar cell.

11. Ornamental element (7) according to at least one of the preceding claims, **characterised in that:**
(i) the components (a), (b) and (c) of the ornamental element (7) are connected by means of an adhesive (5); or
(ii) the components (a), (b) and (c) of the ornamental element (7) are connected by means of an adhesive (5), and the refractive index of the adhesive (5) deviates by less than ± 20% from the refractive index of the gemstone (1).

12. Portable electronic device containing at least one ornamental element (7) according to at least one of the preceding claims 1 to 11.

13. Use of the ornamental element (7) according to at least one of the preceding claims 1 to 11 as an energy source, in particular in portable electronic devices.

## Revendications

1. Élément décoratif (7) contenant
(a) une pierre décorative (1) transparente comportant une surface à facettes comprenant des zones de courbure convexe, et un côté plan ou concave opposé à la surface à facettes,
(b) une couche (3, 4) à sélectivité des longueurs d'ondes, laquelle est appliquée sur le côté opposé à la surface à facettes, et
(c) une cellule (2) photovoltaïque ;
dans lequel la couche (3, 4) à électivité des longueurs d'ondes se trouve entre la pierre décorative (1) et la cellule (2) photovoltaïque, **caractérisé en ce que** la couche (3, 4) à électivité des longueurs d'ondes, dans un intervalle de réflectance large de 50 à 250 nm, lequel est situé dans la plage comprise entre 380 et 850 nm, reflète la lumière incidente à au moins 50 % et que la couche (3, 4) à électivité des longueurs d'ondes présente, à l'extérieur de l'intervalle de réflectance, une transmittance moyenne supérieure à 80 % dans la plage comprise entre 400 et 1 200 nm, mesurée à un angle d'incidence des rayons lumineux de 0°.

2. Élément décoratif (7) selon la revendication 1, **caractérisé en ce que** la pierre décorative (1) est en verre ou en matière plastique.

3. Élément décoratif (7) selon au moins l'une quelconque des revendications précédentes, **caractérisé en ce que** la pierre décorative (1) présente une géométrie plan-convexe ou une géométrie plan-convexe-concave.

4. Élément décoratif (7) selon au moins l'une quelconque des revendications précédentes, **caractérisé en ce que** la couche (3, 4) à électivité des longueurs d'ondes est choisie entre un revêtement (3) à électivité des longueurs d'ondes ou un film (4) à électivité des longueurs d'ondes.

5. Élément décoratif (7) selon la revendication 4, **caractérisé en ce que** le revêtement (3, 4) à électivité des longueurs d'ondes contient au moins un métal et/ou un composé métallique.

6. Élément décoratif (7) selon la revendication 4 ou 5, **caractérisé en ce que** le revêtement (3, 4) à électivité des longueurs d'ondes comprend au moins un composé, lequel est choisi dans le groupe constitué par Cr, Cr₂O₃, Ni, NiCr, Fe, Fe₂O₃, Al, Al₂O₃, Au, SiOₓ, Mn, Si, Si₃N₄, TiOₓ, Cu, Ag, Ti, CeF₃, MgF₂, Nb₂O₅, Ta₂O₅, SnO₂, ZnO₂, MgO, CeO₂, WO₃, Pr₂O₃, Y_{z}O₃, BaF₂, CaF₂, LaF₃, NdF₃, YF₃, ZrO₂, HfO₂, ZnS, des oxynitrures d'AI, de Si et de SnZnO ou toute combinaison desdits composés dans n'importe quelle séquence de couches.

7. Élément décoratif (7) selon au moins l'une quelconque des revendications précédentes 4 à 6, **caractérisé en ce que** le revêtement (3, 4) à électivité des longueurs d'ondes :
(a) comporte une séquence multiple de couches de TiO₂ et de SiO₂ ; ou
(b) consiste en une séquence alternée de TiO₂ et de SiO₂ en douze couches et épaisseurs de couche, lesquelles varient entre environ 20 à 145 nm.

8. Élément décoratif (7) selon au moins l'une quelconque des revendications précédentes 1 à 4, **caractérisé en ce que** la couche (3, 4) à électivité des longueurs d'ondes est un film à électivité des longueurs d'ondes, lequel est appliqué sur le côté de la pierre décorative (1) opposé au côté à facettes et sur la cellule (2) photovoltaïque.

9. Élément décoratif (7) selon au moins l'une quelconque des revendications précédentes, **caractérisé en ce que** la couche (3, 4) à électivité des longueurs d'ondes réfléchit une partie de la lumière dans la plage comprise entre 380 et 850 nm.

10. Élément décoratif (7) selon au moins l'une quelconque des revendications précédentes, **caractérisé en ce que** la cellule (2) photovoltaïque est une cellule solaire à contact par la face arrière.

11. Élément décoratif (7) selon au moins l'une quelconque des revendications précédentes, **caractérisé en ce que** :
(i) les composants (a), (b) et (c) de l'élément décoratif (7) sont liés au moyen d'un adhésif (5) ; ou
(ii) les composants (a), (b) et (c) de l'élément décoratif (7) sont liés au moyen d'un adhésif (5), dans lequel l'indice de réfraction de l'adhésif (5) s'écarte de moins de ±20 % de l'indice de réfraction de la pierre décorative (1).

12. Appareil électronique portable comportant au moins un élément décoratif (7) selon au moins l'une quelconque des revendications précédentes 1 à 11.

13. Utilisation de l'élément décoratif (7) selon au moins l'une quelconque des revendications précédentes 1 à 11 comme source d'énergie, en particulier dans des appareils électroniques portables.
